# EUROPEAN PATENT SPECIFICATION

(11) **EP 1 634 563 B1**
(45) Date of publication and mention of the grant of the patent: **16.11.2011**
(21) Application number: 04021285.4
(22) Date of filing: 07.09.2004
(51) Int. Cl.: A61K 6/083

(54) **Dental polyalkenoate cement composition**
Polyalkenoat-Zahnzementzusammensetzung
Composition de ciment dentaire à base de polyalcenoate

(43) Date of publication of application: 15.03.2006
(73) Proprietor: S & C Polymer Silicon- und Composite-Spezialitäten GmbH, 25335 Elmshorn (DE)
(72) Inventor: Engelbrecht, Jürgen, 25335 Elmshorn (DE); Akinmade, Ademola Olaseni, 25335 Elmshorn (DE); Görlich, Karl-J. Dr, 25335 Elmshorn (DE)
(74) Representative: Forstmeyer, Dietmar

(56) References cited:
- WO-A-97/36943

## Description

The present invention relates to a dental polyalkenoate cement composition to be used for dental restoration or prevention of dental decay. The dental polyalkenoate cement composition according to the present invention especially contains at least one Riboflavin or derivative as a " light-to-heat transformer".

In general, dental polyalkenoate cements are known per se cf. Acid-base cements, their biochemical and industrial applications, A. D. Wilson and J. W. Nicholson, Chemistry of Solid State Materials 3, Cambridge University Press, UK, 1993. They are used in a number of dental applications, including for example: as luting cements for the cementation of dental prostheses; crowns, inlays and bridges to teeth or for the cementation of orthodontic appliances to teeth; as filling cement for the restoration of dental cavities; as sealant cements for sealing pits and fissures in teeth; as luting cements for lining cavities; and as cement for core build-up.

The dental polyalkenoate cement belongs to a group of dental materials that exhibit good biocompatibility and chemical adhesive properties to tooth structure. In particular the dental glass-ionomer cement, a sub-set of polyalkenoate cements, is tooth-like, i.e. the translucency and pigmentation can be made to closely match that of natural tooth. Added to this is the well-known phenomenon of long-term, sustainable fluoride release from glass-ionomer cements. In fact, when the facts are considered and the properties of polyalkenoate cements are evaluated, they have the potential to be excellent dental materials for a wide range of dental remedies and preventive therapies. However, there are a number of limitations that prevent dental polyalkenoate cements from realizing their clinical potential. The features, benefits and limitations of polyalkenoate cements can be summarized in terms of their variants: i) the Classical water only-based polyalkenoate cement; and ii) the resin-modified glass-ionomer cement.

The "Classical" dental polyalkenoate cement comprises finely ground fluoro- aluminosilicate glass powder (or basic oxides), polyalkenoic acid(s) and water. The formation of the water-insoluble dental polyalkenoate cement from this water-based formulation takes place over a number of steps and over a period of time. In summary, in the presence of water, the polyalkenoic acid(s) attack the fluoro-aluminosilicate powder at their acid-susceptible sites and as a result liberate out ions (alkali metal ions, alkaline earth metal ions, and aluminum ions). These liberated metal ions undergo ionic bonding to alkenoic groups of the polyalkenoic acid(s) to form a cross linking structure. In this way the cement "salt" is gelled into a water-insoluble hard mass of material. The realization of this water-insoluble set polyalkenoate cement takes place rapidly over the initial several minutes from the start of the cement mix and then slower over several further hours and days. During this time the cement is susceptible to saliva attack.

A notable and suscessful attempt to improve the properties of the "Classical" glass-ionomer cement led to the development of resin-modified glass-ionomer cements [US Patent No. 4,872,936 Oct 1989]. In these cements, mainly hydrophilic resin monomers with pendant polymerisable acid groups have been introduced into the formulation of the "Classical" cement variant. In so doing, these polymerizable monomers can undergo light and/or chemically-induced polymerization in combination with the "Classical" glass-ionomer acid-base reaction. These resin-modified glass-ionomer cements are improvements on their "Classical" counterparts in terms of mechanical strengths, such as flexural strength and they also have better aesthetics, i.e. lower opacities.

Since dental glass-ionomer cements undergo time-dependent (acid-base ionomer) setting reactions, a certain length of time does elapse before the initial clinical set is attained. Of course, further clinical procedures are held up during this setting stage and within this time dental glass-ionomer cements, especially the Classical variant, are susceptible to saliva attack, as already pointed out. Saliva attack during initial setting is the process of metal ions' elution from the surface of dental glass-ionomer cement restorations due to the action of water from saliva. In this deleterious process, the water content of the cement increases and its metal ion-based cross-linking setting decreases. This results in more opaque restorations with lower surface hardness and, as a consequence, worse clinical wear and performance. In order to overcome this phenomenon, resin-based and other types of water-repellent varnishes are applied to the surfaces of freshly made glass-ionomer restorations. The efficacy of this practice has been reported to vary widely.

A number of attempts have been made to solve this Classical glass-ionomer problem of sluggish set leading to early susceptibility of external water attack. Notable amongst these attempts are 2 citations on the use of infrared energy to hasten the set of glass-ionomer cements [UK Patent No. GB 2 351 086 of January 1999] and [US patent No. 6,264,472 of August 2000]. Another approach that has been proposed [UK Patent No. GB 2357773 A of October 2000], is the addition of coloring matter to dental glass-ionomer cements to reduce their L* values (from normally high values in commercial products) to below 60 and to irradiate such formulations with light in order to hasten their set. A disadvantage of this solution is that formulations with low L* values are dark and a deviation from the desirable tooth-like aesthetics of dental restorative products, including glass-ionomer cements. However, they are acceptable in dental applications with reduced visibility and where colour differentiations may be desirable.

WO 97/36943 describes an elastomeric material obtainable by curing a composition comprising a mixture of a liquid precursor of a glass ionomer cement and a powdered precursor of a glass ionomer cement wherein said liquid precursor comprises at least one polymerisable monomer present in a range of between 2 to 50 % by weight of said liquid precursor of a glass ionomer cement, a carboxylic acid polymer, and an aqueous solvent, wherein said liquid precursor of a glass ionomer cement and said powdered precursor of a glass ionomer cement are present in a ratio of between about 2.5:1 and about 1:1 by weight. This elastomeric material can be prepared by light activated radical polymerisation using camphorquinone, azobisisobutyronitrile or riboflavin as free radical initiator.

The object of this invention is to present dental cements, which when irradiated with radiant energy set faster and possess the attendant benefits thereof. This is irrespective of L* values which in all our examples have remained above 60, due to the addition of Riboflavin or derivatives.

This object is solved by a cement as disclosed in the claims. In particular, this object is solved by providing a dental cement composition containing Riboflavin or derivatives thereof preferably in an amount from 0.001 to 10 % by weight based on the whole cement composition, and more preferred from 0.01 - 1 % by weight. Riboflavin is able to absorb radiant energy within the range of the microwave, infrared, visible and/or ultraviolet spectrum, and to transform this energy into heat. Also the derivatives are able to absorb radiant energy within that range.

When radiant energy is shone on an inventive cement its temperature rises to the benefit of relatively disadvantageous polyalkenoate cement features, such as: slow speed of set; susceptibility to early water solubility; sluggish attainment of hardness; sluggish attainment of bond strength (cohesive strength), etc. Therefore, the above-mentioned inventive cements, upon radiant energy irradiation, improve the clinical performance of a wide range of dental polyalkenoate cements products, such as: sealants; orthodontic and other specialty adhesives; and general restoratives, especially in situations of low saliva management, e.g. in paedriatic and geriatric usage.

In this invention, the term "radiant energy" includes electromagnetic radiation (from any energy source) in the region of microwave, infrared, visible and ultraviolet regions of the electromagnetic spectrum. Preferable is the use of light having a wavelength from 300 to 800 nm, more preferably from 320 to 780 nm, as widely used in current dental remedy.

The temperature of the dental cement composition according to the present invention increases upon irradiation e.g. with light, and the time of initial setting is accelerated, whereby the time-dependent cement properties, such as susceptibility to attack by external water like saliva, hardness and cohesive strength development, etc. are improved.

The dental cement composition of the present invention comprises a fluoro-aluminosilicate glass or basic oxides thereof. These compounds are preferably used as a powder which - in a preferred embodiment - can be finally ground.

As the fluoro aluminosilicate glasses fluoro aluminosilicate glass powders and the like, which are generally used for dental glass-ionomer cements, are preferred. Of these, such fluoro aluminosilicate glass powders are preferred which have as the main components from 10 to 25 % by weight of Al, from 5 to 30 % by weight of Si, from 1 to 30 % by weight of F, from 0 to 20 % by weight of Sr, from 0 to 20 % by weight of Ca, and from 0 to 10 % by weight of alkali metal ions (e.g., Na, Ketc.), based on the whole weight of the glass. Such glasses can be prepared by mixing raw materials containing these components and melting the mixture, and then cooling and pulverizing so as to have a mean particle size of from about 0.02 to 20 pm.

Of the basic oxide variant of the water-based polyalkenoate cement according to the present invention, preferable is the zinc oxide and deactivated zinc oxide/magnesium oxides of the zinc polycarboxylate cements. Preferred also are the newer zinc-based dental compositions [cf. European Patent No. EP0883586]. Other less preferred basic oxide cements include those prepared from Be, Zn, Cu, Mg, Ca, Sr and Ba and combinations thereof.

The vitamin B, especially Riboflavin, or their derivatives may be contained in any of the respective components of the cement: the fluoro-aluminosilicate glass powder; the aqueous solution of polyalkenoic acid(s); or in the components constituting the dental polyalkenoate cement composition in a powder, liquid or paste form.

Polyacids as used in the present invention are polyalkenoic acids. Polyalkenoic acids and their use in dental cements are known per se. They are for example, described in Werner Kullmann, Atlas der Zahnerhaltung, Hanser Verlag, München (1990).

The dental polyalkenoate cement compositions according to the present invention will be hereunder described in more detail with reference to the following glass-ionomer cement Examples, but it is to be construed that the present invention should not be limited thereto.

Example 1. The Effect of the concentration of Riboflavin or vitamin B or their derivatives on the temperature rise in Classical Glass-ionomer Cements

| **Liquid** | **% Riboflavin 5'-mono phosphate, sodium salt dihydrate** | **Temperature Rise, °C** | **Setting Time [Sec]** |
|---|---|---|---|
| Ref Liquid* | 0 | 0.6 | 240 |
| Liquid - 1 | 0.0083 | 1.7 | 236 |
| Liquid -2 | 0.017 | 2.7 | 230 |
| Liquid -3 | 0.04 | 3.3 | 220 |
| Liquid -4 | 0.08 | 4.3 | 210 |

| | | | |
|---|---|---|---|
| *Ref Liquid is the commercial product Vitro Molar Liquid from DFL, RJ Brasil (a classical glass-ionomer cement product). Liquids 1-4 were made by additions of Riboflavin to this product. The cements were formed by mixing the Vitro Molar powder with the liquid at a ratio of 1,4:1. The radiant energy source employed in all of the examples presented was Power Pac manufactured by American Medical Technologies (AMT), Corpus Cristi, USA. | | | |

It can be concluded:
1. Riboflavin 5'- mono phosphate, sodium salt dihydrate increased the setting exotherm of the cements and in so doing made them set faster.
Example 2 (Reference example). The Effect of Resin and Riboflavin or vitamin B or their derivatives inclusion on the temperature rise and setting properties of Glass-ionomer Cements

| **Liquid Constituents** | **Weight % of Constituents** | | | | |
|---|---|---|---|---|---|
| | Liquid-5 | Liquid-6 | Liquid-7 | Liquid-8 | Liquid-9 |
| Riboflavin 5'- mono phosphate, sodium salt dihydrate | 0.08 | 0.08 | 0.08 | 0.08 | 0.0 |
| RM Lute liquid* | 0 | 3.3 | 6.6 | 12.5 | 12.5 |
| Vitro Molar Liquid | 99.9 | 96.7 | 93.4 | 87.5 | 87.5 |
| | 100 | 100 | 100 | 100 | 100 |
| Temperature rise °C | 4.3 | 4.8 | 10.3 | 12.2 | 0.6 |
| Setting Time without light activation [sec] | 240 | 240 | 240 | 240 | 240 |
| Setting Time with 40s radiant energy irradiation [sec] | 210 | 190 | 155 | 120 | 240 |
| Decrease in Setting Time with 40s energy irradiation [sec] | 30 | 50 | 85 | 120 | 0 |
| Decrease in Setting Time with 40s energy irradiation [%] | 12.5 | 20.8 | 35.4 | 50.0 | 0.0 |

| | | | | | |
|---|---|---|---|---|---|
| *RM Lute is a non light-cure resin-modified glass-ionomer commercial product of Strrouf Pharmaceuticals India. The cements were formed by mixing Vitro Molar powder with the liquids at powder: liquid of 1,4:1. | | | | | |

It can be concluded:
1. Riboflavin 5'- mono phosphate, sodium salt dihydrate has the effect of significantly raising the temperature of the polyalkenoate cements.
2. In the absence of Riboflavin 5'- mono phosphate, sodium salt dihydrate, classical and resin-modified polyalkenoate cements have very low temperature rise when irradiated with the energy source.
3. The inclusion of Riboflavin 5'- mono phosphate, sodium salt dihydrate and especially in conjunction with resins has the effect of creating novel polyalkenoate cements with command-cure properties similar in some respect to those of traditional commercial resin-modified polyalkenoate cements.

Examples 1 and 2 have outlined the temperature rise feature and the benefit this has in enabling cements to set faster. Other potential benefits of these Riboflavin-containing products and the proposed curing method have been evaluated and are described following:

Example 3 (Reference example). Increase in Early Surface Hardness - of potential benefit for fissure sealing applications and as an alternative to the varnishing of dental polyalkenoate restorations.

| **Time interval** | **Shore-D Hardness*** | |
|---|---|---|
| | **Without energy irradiation** | **With 40 s energy irradiation** |
| 5 mins from start of mix | 0 | 4 |
| 10 mins from start of mix | 20 | 28 |
| 25 mins from start of mix | 50 | 62 |
| 60 mins from start of mix | 80 | 80 |

The cement of this experiment was Liquid-8 mixed with the Vitro Molar powder, mixed at powder: liquid ratio of 1,4:1 and irradiated with radiant energy for 40s.

Example 4 (Reference example). Development of early high bond strength to un-etched enamel - of potential benefit in the bonding of orthodontic and other dental appliances

| **Time Interval** | **Shear Bond Strength** | |
|---|---|---|
| | **Without light irradiation** | **With 40 s energy irradiation** |
| 30 mins ± 5 mins | 0 MPa | 2.4 MPa |
| 3 hours ± 15 mins | 2.4 MPa | 7.4 MPa |

Liquid 7 was mixed with Vitro Molar powder at powder: liquid ratio of 1.4:1 and applied to poly(acrylic acid) conditioned enamel and irradiated with radiant energy for 40s.

Example 5 (Reference example). The reduction in early water solubility of glass-ionomers with Riboflavin or its derivates or vitamin B

| Time Interval | **Total Dissolved Solids, mg/l** | |
|---|---|---|
| | Without light irradiation | With 40s light irradiation |
| 5.0 mins | 0.083 | 0.074 |
| 5.5 mins | 0.108 | 0.086 |
| 6.0 mins | 0.124 | 0.088 |
| 6.5 mins | 0.143 | 0.108 |
| 7.0 mins | 0.172 | 0.116 |
| 7.5 mins | 0.178 | 0.137 |
| 8.0 mins | 0.184 | 0.148 |
| 8.5 mins | 0.192 | 0.152 |

| | | |
|---|---|---|
| * Liquid 7 was mixed with Vitro Molar powder at powder: liquid ratio of 1.4:1 and 8.0 mg of the cement, 1 mm high was adhered to the bottom of glass bottle with 20 g of distilled water poured over it at t=4,0 minutes from the onset of mix. Energy irradiation was carried out for 40s between t = 3,0 - 4,0 minutes. | | |

## Claims

1. A dental polyalkenoate cement composition
consisting of
- at least one riboflavin or derivatives thereof which are able to absorb radiant energy of a wavelength from 300 to 800 nm, and to transform this light into heat,
- at least one fluoro-aluminosilicate glass powder or basic oxides thereof,
- polyalkenoic acids and
- water.

2. The dental cement composition according to claim 1 wherein the riboflavin and/or its derivative are contained, based on the whole composition, in an amount from 0,001% to 10% by weight.

3. The dental cement composition according to claim 1 or 2 wherein the riboflavin and/or its derivative are contained, based on the whole composition, in an amount from 0,01% to 1% by weight.

## Patentansprüche

1. Polyalkenoat-Zahnzementzusammensetzung, bestehend aus
- mindestens einem Riboflavin oder Derivaten desselben, welche in der Lage sind, Strahlungsenergie einer Wellenlänge von 300 bis 800 nm zu absorbieren, und dieses Licht in Wärme zu transformieren,
- mindestens einem Fluor-Aluminiumsilikatglas-Pulver oder basischen Oxiden desselben,
- Polyalkensäuren und
- Wasser

2. Die Polyalkenoat-Zahnzementzusammensetzung nach Anspruch 1, wobei das Riboflavin und/oder dessen Derivat in einer Menge von 0,001 Gew.-% bis 10 Gew.-%, bezogen auf die gesamte Zusammensetzung, enthalten ist/sind.

3. Die Polyalkenoat-Zahnzementzusammensetzung nach Anspruch 1 oder 2, wobei das Riboflavin und/oder desse Derivate in einer Menge von 0,01 Gew.-% bis 1 Gew.-%, bezogen auf die gesamte Zusammensetzung, enthalten ist/sind.

## Revendications

1. Composition de ciment dentaire à base de polyalcénoate se composant
- d'au moins de la riboflavine ou de ses dérivés qui peuvent absorber l'énergie rayonnante d'une longueur d'onde de 300 à 800 nm, et transformer cette lumière en chaleur,
- d'au moins une poudre de verre de fluoro-aluminosilicate ou ses oxydes basiques,
- d'acides polyalcénoïques et
- de l'eau.

2. Composition de ciment dentaire selon la revendication 1 dans laquelle la riboflavine et/ou son dérivé est/sont contenus, sur la base de l'ensemble de la composition, en une quantité allant de 0,001% à 10% en poids.

3. Composition de ciment dentaire selon la revendication 1 ou 2 dans laquelle la ribovlafine et/ou son dérivé est/sont contenus, sur la base de l'ensemble de la composition, en une quantité allant de 0,01% à 1% en poids.
